# EUROPÄISCHE PATENTSCHRIFT

(11) **EP 1 725 208 B1**
(45) Veröffentlichungstag und Bekanntmachung des Hinweises auf die Patenterteilung: **19.09.2012**
(21) Anmeldenummer: 05706206.9
(22) Anmeldetag: 03.03.2005
(51) Int. Cl.: A61J 7/00, B65B 5/10

(54) **AUTOMATISCHES TABLETTENABFÜLLVERFAHREN UND - SYSTEM**
METHOD AND SYSTEM FOR AUTOMATICALLY FILLING A CONTAINER WITH TABLETS
PROCEDE ET SYSTEME DE REMPLISSAGE AUTOMATIQUE DE COMPRIMES

(30) Priorität: 11.03.2004 AT 4292004
(43) Veröffentlichungstag der Anmeldung: 29.11.2006
(73) Patentinhaber: Knapp AG, 8075 Hart bei Graz (AT)
(72) Erfinder: FREUDELSPERGER, Karl, 8075 Hart bei Graz (AT)
(74) Vertreter: Margotti, Herwig Franz
(86) Internationale Anmeldenummer: PCT/AT2005/000067
(87) Internationale Veröffentlichungsnummer: WO 2005/087175

(56) Entgegenhaltungen:
- US-A- 5 348 061
- US-B1- 6 318 051
- US-B1- 6 449 921
- US-E1- R E37 829

## Beschreibung

Die Erfindung betrifft ein Verfahren und ein System zum automatischen Abfüllen von Tabletten in Tablettenbehälter, die zumindest ein Tablettenfach zur Aufnahme von Tabletten aufweisen.

Aus dem wiederveröffentlichten US-Patent RE37,829 sind ein System und ein Verfahren zum automatisierten Befüllen von Tablettenbehältern mit Tabletten gemäß einem ausgestellten Rezept bekannt. Dieses bekannte Tablettcnabfüllsystem umfasst die folgenden Verarbeitungsmittel :
a) Mittel zur Entgegennahme eines Patientenauftrages, der zumindest ein Rezept und Patienteninformation enthält;
b) Zuordnungsmittel zum Zuordnen der Rezepte zu automatischen Tabletten-Abfülllinien, die wiederum umfassen:
c) Tablettenabfüllmittel zum Abfüllen von Tabletten in verschließbare Tablettenbehälter gemäß dem Rezept, wobei die Tabletten aus einer Vielzahl unterschiedlicher Tablettensorten auswählbar sind und jede Tablettensorte in einen separaten Tablettenbehälter abgefüllt wird
d) Etikettiermittel zum Etikettieren der Tablettenbehälter, wobei auf den Etiketten der Name der Tablettensorte und gegebenenfalls eine Einnahmevorschrift enthalten ist;
e) Kappenaufsetzmittel zum Verschließen der Tablettenbehälter;
f) Tablettenbehälter-Sortiermittel zum automatischen Sortieren der befüllten Tablettenbehälter gemäß dem Patientenauftrag, und
g) Sammelmittel, um automatisch Tablettenbehälter zusammenzufassen, die zu einem Patientenauftrag gehören;
g) Transportmittel zum Transportieren der Tablettenbehälter zu den einzelnen Verarbeitungsmitteln.

Dieses System zum Befüllen von Tablettenbehältern mit Tabletten gemäß einem ausgestellten Rezept hat sich bei Großapotheken bewährt, wo die einzelnen Tablettensorten nicht in Kleinmengen in einzelnen Schachteln vorverpackt gelagert werden, sondern unverpackt in Großbehältern gelagert werden, aus denen dann nach Rezept die erforderliche Stückzahl abgezählt und abgegeben wird.

Obgleich durch das bekannte System die Aufgabe der Aufteilung von Tablettensorten in einzelne Tablettenbehälter zufriedenstellend gelöst ist, so hat sich gezeigt, dass speziell für ältere Personen oder Personen mit chronischen Erkrankungen die Einnahme von Medikamenten aus einzelnen Tablettenbehältern ein durchaus ernst zu nehmendes Problem darstellt, da sie meist aus einer Vielzahl von Medikamenten zur richtigen Tageszeit die richtige Rezeptur selbst zusammenstellen müssen, womit viele Patienten aufgrund von Altersvergesslichkeit oder anderer körperlicher und geistiger Beeinträchtigungen aufgrund ihres Krankheitsbildes überfordert sind. Für Patienten in Pflegeheimen kann diese Versorgung mit Medikamenten vom Pflegepersonal sichergestellt werden. Patienten, die sich in häuslicher Pflege befinden, sind jedoch dabei auf sich allein gestellt. Studien besagen, dass ca. 75 % der verordneten Medikamente nicht nach Vorschrift eingenommen werden und bestätigen damit das geschilderte Problem. Ebenso werden die verordneten Medikamente nicht immer bis zur Gänze aufgebraucht, bevor neue Medikamentenpackungen bezogen werden. Es besteht daher aus medizinischen und volkswirtschaftlichen Gründen ein dringendes Bedürfnis zur Lösung dieses Problems.

Diese Problem der Unfähigkeit vieler Patienten die ihnen verordneten Medikamente zur richtigen Zeit und in der richtigen Dosierung einzunehmen, wurde bereits von Apothekern erkannt. Daher bieten Apotheker als Kundendienst solchen Patienten an, Wochenrationen der Medikamente in Blisterpackungen abzufüllen, die vom Patienten in der Apotheke abgeholt werden können. Solche Blisterpackungen sind üblicherweise in sieben Wochentage unterteilt. Für jeden Wochentag sind weitere Unterteilungen (morgens, mittags, abends und nachts) vorgesehen. Diese Blisterpackungen werden manuell mit den für den Patienten vorbestimmten Medikamenten befüllt. Anschließend wird der Blister versiegelt und mit patientenspezifischen Daten versehen. Allerdings ist dieses manuelle Abfüllen sehr zeit- und damit kostenaufwändig und stellt für die damit befassten Personen eine hohe Belastung dar, da die Tätigkeit einerseits äußerst monoton und andererseits gleichzeitig extrem verantwortungsvoll ist, indem Fehlbefüllungen schwere gesundheitliche Schädigungen des Patienten nach sich ziehen können. Weiters ist es schwierig, beim manuellen Abfüllen gewisse arzneirechtliche, Sicherheits- und Kontrollstandards einzuhalten.

Aus diesen Gründen besteht nach wie vor das Bedürfnis nach einem automatisierten System zum Befüllen von Tablettenbehältern mit unterschiedlichen Tablettensorten in vorgegebener Stückzahl, mit dem bereits der Arzneimittelproduzent oder Arzneimittel-Großhändler in der Lage ist, diese Tablettenbehälter zu befüllen. Als Vertriebsnetz sind dabei Apotheken vorgesehen, die die fertig befüllten Tablettenbehälter an die Patienten abgeben.

Die vorliegende Erfindung löst die geschilderten Probleme durch Bereitstellen eines Verfahrens zum automatischen Abfüllen von Tabletten in Tablettenbehälter mit den Merkmalen des Anspruchs 1, sowie durch Bereitstellen eines Systems zum automatischen Abfüllen von Tabletten in Tablettenbehälter mit den Merkmalen des Anspruchs 11. Vorteilhafte Ausgestaltungen der Erfindung sind in den abhängigen Ansprüche dargelegt.

Durch die vorliegende Erfindung ist es möglich, Tablettenbehälter mit einem oder vorzugsweise mehreren Fächern automatisch so zu füllen, dass in einem Tablettenfach exakt jene Medikation enthalten ist, die der Patient zu einem bestimmten Zeitpunkt einnehmen soll. Vorteilhaft sind die Tablettenbehälter mit sieben Tablettenfächern (für jeden Tag der Woche eines) oder einem Vielfachen von sieben Fächern, falls mehrmals pro Tag Medikamente eingenommen werden müssen, ausgestattet. Vorteilhaft sind die Tablettenbehälter als Blister ausgebildet.

Es sei erwähnt, dass wie hierin verwendet der Begriff "Tablette" jedwedes oral zu verabreichendes festes Medikament, also auch Kapseln und Pillen umfasst.

In einer bevorzugten Ausgestaltung der Erfindung umfasst das Zuordnen der Rezeptdaten und Patienten-Identifikation zu jeweiligen Tablettenbehältern das Übertragen der Rezeptdaten und Patienten-Identifikation auf einen oder mehrere Informationsträger und das Zuordnen eines jeweiligen Informationsträgers zu jeweils einem Tablettenbehälter. Durch diese Maßnahmen besteht zu jedem Zeitpunkt des Tablettenabfüllprozesses eine feste Assoziation zwischen den Patienten- und Rezeptdaten und dem Tablettenbehälter. Der Abfüllvorgang kann somit weitgehend dezentral gesteuert ablaufen; Überprüfungen des Prozessfortschritts und der fehlerfreien Abwicklung sind an jeder Verarbeitungsstation des Tablettenabfüllsystems möglich. Dazu werden die Tablettenbehälter und die ihnen zugeordneten Informationsträger gemeinsam durch den zumindest einen Tablettenabgabeautomaten hindurchgefördert, der auf dem Informationsträger gespeicherte Rezeptdaten ausliest, die von dem Patienten zu jeweiligen Zeitpunkten einzunehmende Anzahl an Tabletten der jeweiligen Tablettensorten ermittelt und jeden ermittelten Tabletten-Einnahmezeitpunkt zu einem Tablettenfach zuordnet und die jeweils erforderliche Anzahl an Tabletten in das jeweilige zugeordnete Tablettenfach abfüllt. Somit wird der Datenverkehr zu einem übergeordneten Host-Steuersystem deutlich reduziert. Als Informationsträger sind, vorzugsweise berührungslos, beschreibbare und auslesbare elektronische Datenträger, oder bedruckbare Substrate, wie ein Barcodeetiketten, vorgesehen.

Zum Wohle des Patienten ist es zweckmäßig, wenn bereits beim Empfangen des Patientenauftrages eine Plausibilitätskontrolle der Rezeptdaten hinsichtlich möglicher Überdosierung und gegenseitiger Verträglichkeit von Tablettensorten durchgeführt und bei Erkennen von Überdosierung oder Unverträglichkeit der Patientenauftrag zurückgewiesen wird.

Weiters wird für den Patienten die ordnungsgemäße Einnahme der verschriebenen Tabletten wesentlich erleichtert, wenn die Tablettenbehälter automatisch mit aus den Rezeptdaten abgeleiteter Information, wie den Einnahmevorschriften versehen wird. Für die Auslieferung des befüllten Tablettenbehälters an den Patienten ist es zweckmäßig, wenn der Tablettenbehälter mit aus der Patienten-Identifikation abgeleiteter Information, wie Namen und Adresse versehen wird, vorzugsweise durch Bedrucken oder Etikettieren des Tablettenbehälters.

Um jedes Risiko für den Patienten zu vermeiden, ist in einer Ausgestaltung der Erfindung vorgesehen, dass beim Auftreten von Fehlern bei der Durchführung des Tablettenabfüllverfahrens für einen Tablettenbehälter das Tablettenabfüllverfahren abgebrochen und der Tablettenbehälter ausgeschieden wird. Dabei ist es zweckmäßig, wenn beim Auftreten von Problemen oder Fehlern beim Abarbeiten des Tablettenabfüllverfahrens Fehlerkennzeichnungen auf die Informationsträger der gerade bearbeiteten Tablettenbehälter geschrieben werden und mit Fehlerkennzeichnungen gekennzeichnete Tablettenbehälter zentral am Ende des Abfüllverfahrens ausgeschieden werden. Um zu verhindern, dass bereits als fehlerbehaftet gekennzeichnete Tablettenbehälter weiter mit Tabletten befüllt werden, ist es zweckmäßig, wenn vor jedem Verarbeitungsschritt des Tablettenabfüllverfahrens für jeden Tablettenbehälter überprüft wird, ob der diesem Tablettenbehälter zugeordnete Informationsträger eine Fehlerkennzeichnung enthält und bei Erkennen einer solchen Fehlerkennzeichnung der jeweilige Verarbeitungsschritt nicht durchgeführt wird.

Zur vereinfachten Auslieferung der Tablettenbehälter an einen Patienten ist es vorteilhaft, wenn alle zu einem Patientenauftrag gehörenden Tablettenbehälter automatisch gesammelt werden.

Da die derzeit allgemein verwendeten Tablettenbehälter aus tiefgezogener Kunststofffolie bestehen und mechanisch wenig belastbar sind, ist in einer Fortbildung der Erfindung vorgesehen, für jeden Tablettenbehälter eine Trägereinrichtung bereitzustellen, auf der der Tablettenbehälter durch das Tablettenabfüllsystem hindurch transportierbar ist. Man vermeidet dadurch Beschädigungen des Tablettenbehälters und kann die Verarbeitungsgeschwindigkeit des Tablettenabfüllsystems wesentlich beschleunigen. Die Trägereinrichtung verbleibt im Tablettenabfüllsystem, so dass ihre Herstellungskosten - verglichen mit den Herstellungskosten der Einweg-Tablettenbehälter - weniger zu beachten sind, da sie über lange Zeit verwendet werden können und ihre Anzahl relativ gering ist. Daher ist es auch zweckmäßig, den Informationsträger auf der Trägereinrichtung anzuordnen und somit vielmals verwenden zu können.

Es hat sich zur Erhöhung der Betriebsverlässlichkeit als zweckmäßig erwiesen, die Tablettenautomaten modular aus einer Vielzahl von Tablettenabgabestationen aufzubauen, die jeweils einen Vorrat einer Tablettensorte enthalten und eine einstellbare Anzahl von Tabletten an beliebige Tablettenfächer der Tablettenbehälter abgeben.

In einer leicht wartbaren und nachfüllbaren Ausgestaltung des erfindungsgemäßen Tablettenabfüllsystems umfasst jede Tablettenabgabestation eine Vielzahl von Tablettenabgabeeinheiten, die von einem gemeinsamen Tablettenmagazin versorgt werden, wobei die Tablettenabgabeeinheiten vorzugsweise als Rotationsabgabeeinheiten ausgebildet sind. Das Tablettenmagazin kann weiters mit einem auswechselbaren Pufferbehälter verbindbar sein, wobei verschiedene Vorsichtsmaßnahmen zum Vermeiden von Verwechslungen getroffen werden können, wie z.B. hardwaremäßige Codierungen.

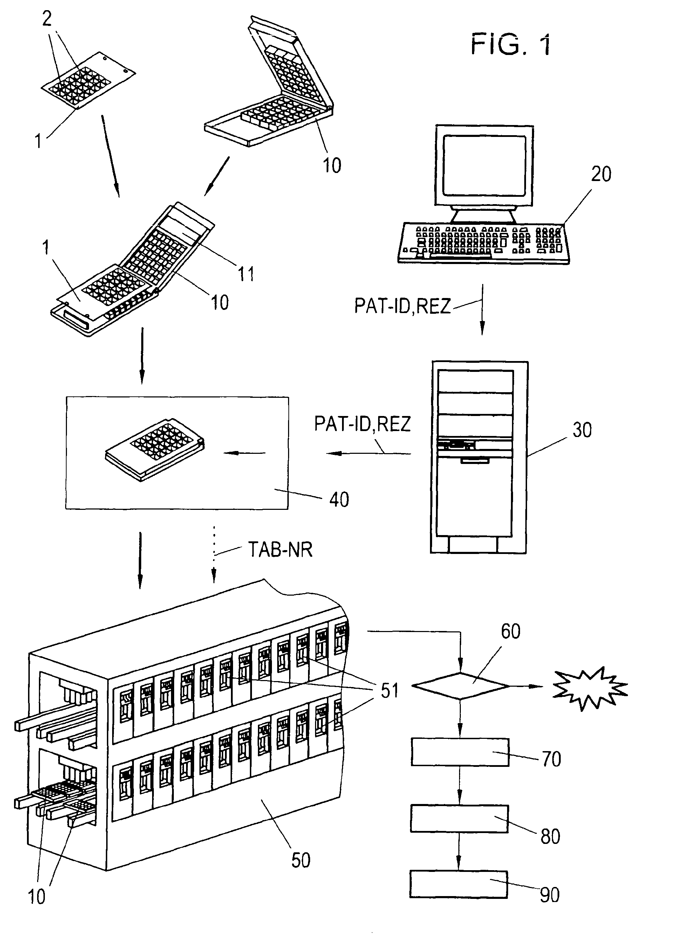

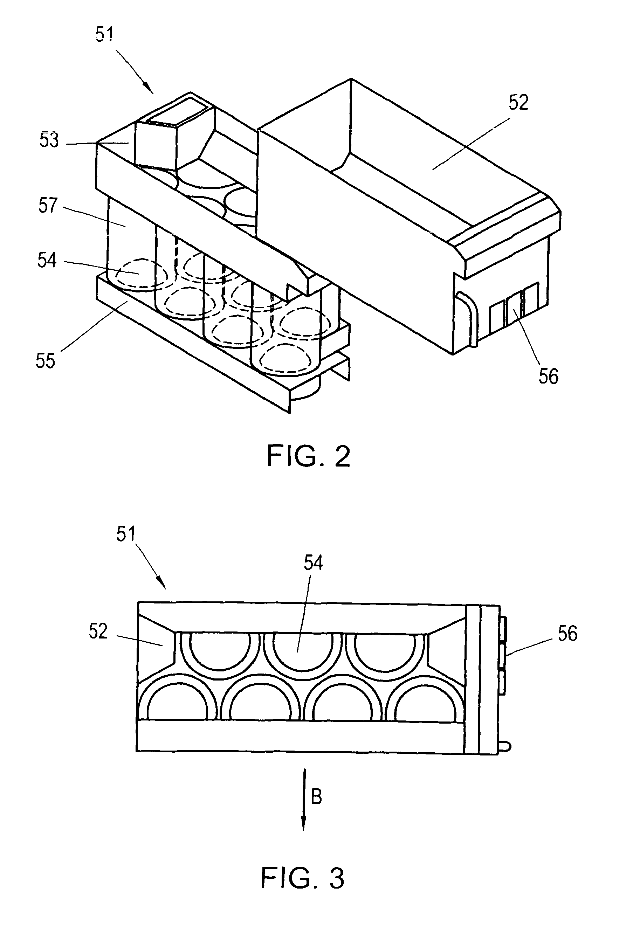

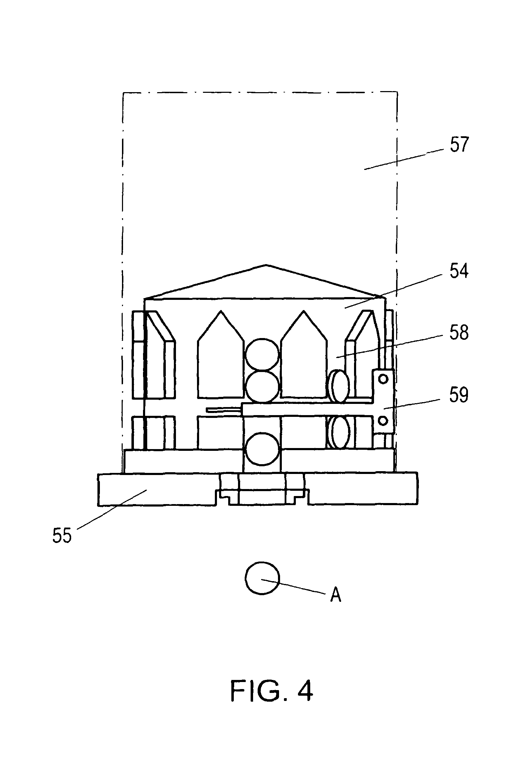

Die Erfindung wird nun anhand eines nicht einschränkenden Ausführungsbeispieles unter Bezugnahme auf die Zeichnungen näher erläutert. In den Zeichnungen zeigen Fig. 1 eine schematische Darstellung des erfindungsgemäßen Tablettenabfüllsystems, Fig. 2 eine Tablettenabgabestation in der Perspektive, Fig. 3 eine Draufsicht der Tablettenabgabestation von Fig. 2, und Fig. 4 eine Seitenansicht einer in der Tablettenabgabestation verwendeten Rotations-Tablettenabgabeeinheit.

Das in Fig. 1 dargestellte erfindungsgemäße Tablettenabfüllsystem dient dazu, von einem Arzt für einen Patienten verschriebene Medikamente automatisch in der vorgesehenen Einzeldosierung in Tablettenfächer eines Tablettenbehälters abzufüllen. Dazu wird die Rezeptdaten REZ zusammen mit Patientenidentifikationsdaten PAT-ID, wie Namen und Adresse des Patienten, entweder direkt vom Arzt in elektronischer Form über einen Computer 20 an Empfangsmittel 30 des erfindungsgemäßen Tablettenabfüllsystems übermittelt, oder ein schriftliches Rezept des Arztes vom Patienten bei einem Apotheker abgegeben, der das Rezept in ein elektronisches Format umwandelt und die Rezeptdaten REZ zusammen mit Patientenidentifikationsdaten PAT-ID mittels eines Computers an die Empfangsmittel 30 des Tablettenabfüllsystems sendet. Die Übermittlung dieser Patientenaufträge an die Empfangsmittel 30 findet kontinuierlich statt. Der Patientenauftrag wird anschließend von den Empfangsmitteln 30 einer FIFO ("first in, first out") Kette angereiht, um in der eingetroffenen Reihenfolge verarbeitet zu werden, d.h. um die verschriebenen Tabletten im Produktionslager in Tablettenbehälter zu kommissionieren, einschließend den Tablettenbehätter zu verschließen, mit kundenspezifischen Daten zu versehen und in einem Versandbereich zur weiteren Verwendung zur Verfügung zu stellen. Es sei erwähnt, dass Rezepte mit dem Status "eilig" gesondert behandelt werden können. Zur Durchführung dieser Verarbeitung geben die Empfangsmittel 30 den Patientenauftrag, d.h. die Patienten-Identifikation PAT-ID und zumindest ein Rezept mit Rezeptdaten REZ über von dem Patienten einzunehmende Tablettensorten und ihre Einnahmevorschriften an Zuordnungsmittel 40 weiter, wo das Rezept REZ und die Patienten-Identifikation PAT-ID zu jeweiligen Tablettenbehältern 1, die eine Vielzahl an Tablettenfächern 2 aufweisen, zugeordnet werden. In einer Variante dieser Ausführungsform ermitteln die Zuordnungsmittel zunächst aus den Rezeptdaten REZ die von einem Patienten zu jeweiligen Zeitpunkten einzunehmende Anzahl TAB-NR an Tabletten der jeweiligen Tablettensorten und ordnen diese Information jeweiligen Tablettenfächern eines Tablettenbehälters zu bzw. geben diese Information an einen nachfolgenden Tablettenabgabeautomaten 50 weiter. In der Grundversion des erfindungsgemäßen Tablettenabfüllsystems ist der Tablettenabgabeautomat 50 jedoch dazu ausgebildet, aus den dem zu befüllenden Tablettenbehälter 1 zugeordneten Rezeptdaten REZ selbst die erforderliche Anzahl an Tabletten und die Tablettenfächer 2, in die die Tabletten zu positionieren sind, zu ermitteln.

Um das erfindungsgemäße Tablettenabfüllsystem unabhängig von zu verwendenden Tablettenbehältern zu halten, bzw. den schlechten Transporteigenschaften üblicher Tablettenbehälter Rechnung zu tragen, wird eine Trägereinrichtung 10 verwendet, wie in Fig. 1 dargestellt. Diese Trägereinrichtung 10 weist ebenfalls eine Vielzahl von Tablettenfächern auf, die in dem Tablettenabgabeautomaten 50 mit den notwendigen Medikamenten befüllt, an einer nachfolgenden Kontrollstation 60 auf Vollständigkeit und Richtigkeit überprüft werden und abschließend der Inhalt der Trägereinrichtung in einer Umfüllstation dem Tablettenbehälter übergeben werden. In einer derzeit bevorzugten Ausgestaltung der Erfindung ist die Trägereinrichtung 10 so ausgebildet, dass sie einen Tablettenbehälter 1 direkt aufnehmen und durch das gesamte Tablettenabfüllsystem transportieren kann. Im dargestellten Ausführungsbeispiel ist der Tablettenbehälter 1 als Blisterpackung ausgebildet. In der nachfolgenden Beschreibung werden daher die Tablettenbehälter 1 auch als Blister bezeichnet und die Trägereinrichtung 10 als Pseudo-Blister. Jeder Blister umfasst 4 x 7 Tablettenfächer 2, d.h. Tablettenfächer für vier Einnahmezeitpunkte an allen Wochentagen. Jeder Blister 1 ist in einen Pseudo-Blister 10 einsetzbar und durch das Zuklappen eines Deckels des Pseudo-Blisters mit 28 Öffnungen, die den Tablettenfächern 2 entsprechen, in fixer Lage arretierbar.

Jeder Pseudo-Blister 10 ist mit einem berührungslos beschreibbaren und lesbaren Transponder als Informationsträger 11 versehen. Die Zuordnungsmittel 40 schreiben die Rezeptdaten REZ und die Patienten-Identifikation PAT-ID auf den Informationsträger 11, so dass diese Informationen auf der gesamten Reise des Pseudo-Blisters 10 durch das Tablettenabfüllsystem jederzeit abfragbar sind. Die Rezeptdaten REZ stellen das Steuerglied im Prozess dar. Zur erleichterten Kontrolle durch eine Bedienperson könnte der Informationsträger 11 auch ein bedruckbares Feld enthalten, auf dem die Rezeptdaten und Patienten-Identifikation in lesbarer Form oder für die Bedienperson verständlicher Codierung aufgedruckt sind. Es sei erwähnt, dass die patientenauftragsspezifischen Daten (Medikament, Anzahl, etc.) einer Plausibilitätskontrolle bezüglich gegenseitiger Verträglichkeit, Überdosierung, Kontraindikationen unterzogen werden können, wobei eine solche Plausibilitätskontrolle zweckmäßig bereits in die Empfangsmittel 30 integriert ist.

An jeder Stelle im erfindungsgemäßen Tablettenabfüllsystem, an denen irgendeine Entscheidung zu treffen ist (=Entscheidungsstationen), befinden sich Transponderreader, die den Inhalt des Informationsträgers 11 auslesen und aufgrund des Leseergebnisses den richtigen Materialfluss sicherstellen. Müssen an einer Station Aktionen ausgeführt werden, werden allfällig auftretende fehlerhafte Ereignisse (Fehlerkennzeichnungen) abschließend auf den Informationsträger 11 geschrieben. Ergebnisse aller Entscheidungsstationen werden additiv einem übergeordneten Steuerrechner übermittelt und langfristig gespeichert. Pseudo-Blister, an denen Fehler aufgetreten sind, z.B. die an Stationen falsch befüllt wurden, werden aus dem Materialfluss entnommen, ihr Inhalt wird entsorgt. Anschließend wird dieser Auftrag erneut gestartet.

Der Tablettenabgabeautomat 50 ist modular aus einer Vielzahl von einzelnen Tablettenabgabestationen 51 aufgebaut, sodass für jede Medikamenten-Produktklasse eine eigenständige Tablettenabgabestationen 51 zum Einsatz kommen kann. Die Tablettenabgabestationen 51 haben die Aufgabe, Pillen aus einem Vorratsbehälter in die richtigen Tablettenfächer 2 eines Tablettenbehälters 1 zu übergeben.

Eine erfindungsgemäße Ausführungsform einer Tablettenabgabestation 51 ist in den Figuren 2 und 3 in der Perspektive bzw. in Draufsicht dargestellt. Die Tablettenabgabestation 51 umfasst eine Vielzahl von Tablettenabgabeeinheiten 54, im vorliegenden Fall sieben, die den sieben Reihen an Wochentags-Tablettenfächern entsprechen. Pfeil B in Figur 3 stellt die Transportrichtung der Tablettenbehälter bzw. der Pseudo-Blister unter der Tablettenabgabestation 51 dar. Der Pseudo-Blister wird so durch den Dispenserautomat getaktet, dass Medikamente für jeweils sieben Wochentage parallel abgegeben werden können. Man erkennt, dass zur Erzielung der dafür notwendigen kompakten Bauweise die Tablettenabgaheeinheiten 54 in zwei gegeneinander versetzten Reihen angeordnet sind. Jede Tablettenabgabeeinheit 54 ist als Rotationsabgabeeinheit ausgebildet und umfasst ein zylindrisches Rohr 57 zur Zuführung der Tabletten von einem allen Tablettenabgabeeinheiten einer Tablettenabgabestation 51 gemeinsamen Tablettenmagazin 53. Das Tablettenmagazin 53 ist wiederum mit einem auswechselbaren Pufferbehälter 52 verbindbar, der einen Übervorrat an Tabletten enthält. Es sind sowohl am Tablettenmagazin 53 als auch am Pufferbehälter 52 Farbcodierungen 56 vorgesehen, um einer Betriebsperson eine einfache Kontrollmöglichkeit zu bieten, ob tatsächlich die richtigen Pufferbehälter eingesetzt werden. Ebenso können hardwaremäßige Codierungen vorgesehen sein, mit denen verhindert wird, dass nicht zugehörige Pufferbehälter in das Tablettenmagazin eingesetzt werden können, wie weiter unten näher erklärt wird. Die Tablettenabgabeeinheit 54 kann als Rotationsabgabeeinheit gemäß dem US Patent Nr. 5,803,309 ausgeführt sein, dessen Offenbarung hiermit durch Verweis aufgenommen wird. Fig. 4 zeigt eine Seitenansicht der Rotationsabgabeeinheit 54 gemäß US 5,803,309. Man erkennt unter dem Tablettenzuführzylinder 57 die eigentliche Rotationseinheit mit Fächern 58 zur säulenförmigen Übereinander-Anordnung von Tabletten A in den Fächern. Ein Trennelement 59 dient dazu nur einzelne Tabletten A über eine Auswurfeinheit 55 nach unten abzugeben, wo sie direkt in ein Tablettenfach des Tablettenbehälters bzw. des Pseudo-Blister fallen.

Die Steuerung des Abfüllvorganges im Tablettenabgabeautomaten 50 erfolgt folgendermaßen. Am als Transponder ausgebildeten Informationsträger 11 des Pseudo-Blisters 10 ist in Form der Rezeptdaten REZ gespeichert bzw. ermittelbar, aus welcher Tablettenabgabestation 51 wie viel zu entnehmen ist. Eine Taktpositionierung stellt sicher, dass die jeweilige Stückzahl eines Medikaments in das richtige "Tageszeiten-Tablettenfach" abgegeben wird. Ein Pseudo-Blister wird für ein Medikament ausschließlich von einer Tablettenabgabestation 51 bedient. Zusätzlich zum Informationsträger ist der Pseudo-Blister hardcodiert. Dadurch kann über Lichttaster die Positionierung des Pseudo-Blisters kontrolliert werden. Während der Pseudo-Blister weitergetaktet wird, wird in der Tablettenabgabestation 51 der nächste Abgabevorgang vorbereitet. Dazu wird über eine Lichtschranke im jeweiligen Rotor 54 kontrolliert, ob ein Medikament im nachfolgenden Fach 58 des Rotors vorhanden ist. Fehlt in diesem Rotorfach 58 das Medikament, wird der Positioniervorgang des Rotors solange fortgesetzt, bis sich ein mit einem Medikament gefülltes Fach unmittelbar vor der Auswurfeinheit 55 befindet. Über eine (nicht dargestellte) Gabellichtschranke wird der Abgabevorgang überwacht. Eine Signalflankenauswertung dieser Gabellichtschranke stellt sicher, dass die richtige Anzahl von Medikamenten dispensiert wurde.

Jeder Dispensionsvorgang wird von der Hardware des Tablettenabgabeautomaten überwacht und kontrolliert. Fehler werden direkt in den Transponder geschrieben und zusätzlich einem übergeordneten Steuerrechner mitgeteilt. Nach jedem Tablettenabgabeautomat befinden sich Kontrollmittel 60, die falsch befüllte oder auf andere Weise fehlerhafte Pseudo-Blister 11 identifizieren und ausscheiden. Der Inhalt dieser ausgeschiedenen Pseudo-Blister wird anschließend recycelt, und der Pseudo-Blister wird wieder zum Startpunkt des Systems transportiert. Aufträge, deren Inhalt falsch kommissioniert wurde, werden nochmals gestartet.

Die Hardware eines Tablettenabgabeautomaten wird nach der Kalibrierung (Zuordnung des abzugebenden Medikaments zu den Abgabestationen) mit Stammdaten beschrieben (Tablettenabgabeautomat-Nummer, Produktname der zu dispensierenden Medikamente, Software-Version, Hardware-Version, etc.).

Der Füllstand der Pufferbehälter 52 wird mit einer Lichtschranke kontrolliert. Ist ein Pufferbehälter 52 leer, muss er ersetzt werden. Damit die betroffene Tablettenabgabestation 51 trotzdem weiterarbeiten kann, ist das Tablettenmagazin 53 vorgesehen. Das Füllvolumen des Tablettenmagazins 53 ist dabei so zu wählen, dass das Nachfüllen des Pufferbehälters ohne Unterbrechung des Abgabevorganges möglich ist. Um Produktvertauschung zu verhindern, ist jeder Puffer einerseits farbcodiert und andererseits hardcodiert. Der Hardware einer Tablettenabgabestation 51 wird über eine Interaktion mitgeteilt, dass ihr Pufferbehälter 52 gerade gewechselt wird. Die Person, die den Nachfüllvorgang ausführt, kann durch die Farbcodierung feststellen, ob der richtige Pufferbehälter aufgesetzt bzw. eingeschoben wurde. Die Hardware erkennt, dass ein neuer Pufferbehälter aufgesetzt bzw. eingeschoben wurde. Jeder Pufferbehälter ist mit einem elektronischen Datenträger versehen, der während des Befüllens des Pufferbehälters mit der Prdduktinformation beschrieben wurde. Diese Information wird nun von der Hardware der Tablettenabgabestation ausgelesen. Stimmen die Daten des Pufferbehälters mit den Stammdaten der Hardware der Tablettenabgabestation überein, wird der Pufferbehälter entriegelt. Danach kann man die mechanische Sperre, die den Pufferbehälter 52 vom Tablettenabgabemagazin 53 trennt, entfernen. Die Medikamente fallen danach in das Tablettenabgabemagazin 53. Jeder Nachfüllvorgang wird mitprotokolliert und dem übergeordneten Steuerungssystem gemeldet.

Das übergeordnete Steuerungssystem ist für "Tracking and Tracing" zuständig. Von jedem Tablettenabgabeautomaten des Tablettenabfüllsystems wird die History in einer Datenbank abgelegt. Zu jedem Zeitpunkt kann somit nachvollzogen werden, welcher Patientenauftrag wann und mit welchen Medikamenten (Charge) ausgestattet wurde.

Leere Pufterbehälter werden an einem bestimmten Arbeitsplatz mit Medikamenten befüllt. Dabei ist darauf zu achten, dass die richtigen Medikamente in die dafür vorgesehenen Pufferbehälter gegeben werden.

Der Abrieb der Medikamente führt dazu, dass nach einer endlichen Anzahl von Tablettenabgaben die Tablettenabgabestation verschmutzt. In Abhängigkeit vom Verschmutzungsgrad nimmt die Qualität des Dispensionsvorgangs ab. Deshalb werden Reinigungsintervalle festgelegt, in denen die Tablettenabgabestationen in einer Art "Waschstrasse" gereinigt werden.

Nachdem die einzelnen Patientenaufträge abgearbeitet wurden, werden von den Kontrollmitteln 60 die Inhalte der befüllten Pseudo-Blister mit Hilfe bildgebender Systeme auf Richtigkeit und Vollständigkeit überprüft. Die Ergebnisse werden einem übergeordneten Steuerungssystem übergeben und zusammen mit den Kundendaten abgespeichert. Im Falle eines negativen Ergebnisses wird der Pseudo-Blister aus dem Materialfluss entnommen und entweder manuell nachkontrolliert oder recycelt, wobei der Auftrag nochmals gestartet wird.

Nach den Kontrollmitteln 60 werden ordnungsgemäß befüllte und fehlerfreie Tablettenbehälter von ihren Trägereinrichtungen getrennt, mittels Verschließmitteln 70 verschlossen und in einer Druckstation 80 mit patientenspezifischen Daten bedruckt.

Fertig verschlossene und bedruckte Tablettenbehälter werden zum Abschluss durch Sammelmittel 90 gesammelt und gegebenenfalls sortiert. Dazu muss jeder Tablettenbehälter maschinenlesbare Patientenidentifikationen, z.B. einen Barcode, aufweisen.

Das übergeordnete Steuerungssystem nimmt die abzuarbeitenden Aufträge von einem HOST-System entgegen. Zusätzlich wird der Materialfluss der Pseudo-Blister sichergestellt und kontrolliert. Jedes Ereignis der einzelnen Pseudo-Blister wird in einer Datenbank aufgezeichnet. Die Ergebnisse der Kontrollstation werden ebenso in dieser Datenbank aufgezeichnet. Ein Leitstand gibt Auskunft über den Zustand des Gesamtsystems. Die Bediener des Tablettenabfüllsystems müssen sich am System anmelden, um Aktivitäten (Nachfüllvorgang, Produktwechsel, Wartung, etc.) durchführen zu dürfen.

## Patentansprüche

1. Verfahren zum automatischen Abfüllen von Tabletten in Tablettenbehälter, die zumindest ein Tablettenfach zur Aufnahme von Tabletten aufweisen, **gekennzeichnet durch**
das Empfangen eines Patientenauftrages, der eine Patienten-Identifikation (PAT-ID) und zumindest ein Rezept mit Rezeptdaten (REZ) über von dem Patienten einzunehmende Tablettensorten und ihre Einnahmevorschriften enthält;
das Zuordnen der Rezeptdaten (REZ) und Patienten-Identifikation (PAT-ID) zu jeweiligen Tablettenbehältern (1);
das automatische Befüllen zumindest eines Tablettenfaches (2) eines jeweiligen Tablettenbehälters (1) **durch** zumindest einen Tablettenabgabeautomaten (50), der Vorräte an einer Vielzahl von Tablettensorten enthält,
wobei aus den Rezeptdaten (REZ) die von einem Patienten zu jeweiligen Zeitpunkten einzunehmende Anzahl an Tabletten der jeweiligen Tablettensorten ermittelt werden und jeder ermittelte Tabletten-Einnahmezeitpunkt einem Tablettenfach (2) eines Tablettenbehälters (1) zugeordnet wird und die so ermittelten Tabletten für jeden ermittelten Einnahmezeitpunkt in das zugeordnete Tablettenfach (2) abgefüllt werden;
das Verschließen und Abgeben der befüllten Tablettenbehälter (1).

2. Tablettenabfüllverfahren nach Anspruch 1, **dadurch gekennzeichnet, dass** das Zuordnen der Rezeptdaten (REZ) und Patienten-Identifikation (PAT-ID) zu jeweiligen Tablettenbehältern (1) das Übertragen der Rezeptdaten und Patienten-Identifikation auf einen oder mehrere Informationsträger (11) und das Zuordnen eines jeweiligen Informationsträgers zu jeweils einem Tablettenbehälter (1) umfasst.

3. Tablettenabfüllverfahren nach Anspruch 2, **dadurch gekennzeichnet, dass** als Informationsträger (11) ein, vorzugsweise berührungslos, beschreibbarer und auslesbarer elektronischer Datenträger, oder ein bedruckbares Substrat, wie ein Barcodeetikett, bereitgestellt wird.

4. Tablettenabfüllverfahren nach Anspruch 2 oder 3, **dadurch gekennzeichnet, dass** das automatische Befüllen zumindest eines Tablettenfaches (2) eines jeweiligen Tablettenbehälters (1) durch zumindest einen Tablettenabgabeautomaten (50) das gemeinsame Fördern der Tablettenbehälter (1) und der ihnen zugeordneten Informationsträger (11) durch den zumindest einen Tablettenabgabeautomaten;
das Auslesen der auf dem Informationsträger (11) gespeicherten Rezeptdaten durch den jeweiligen Tablettenabgabeautomaten (50);
das Überprüfen durch den jeweiligen Tablettenabgabeautomaten (50), ob die in ihm gelagerten Vorräte an Tablettensorten mit einer der in den Rezeptdaten (REZ) enthaltenen Tablettensorten korrespondieren und bei Korrespondenz
das Ermitteln der von dem Patienten zu jeweiligen Zeitpunkten einzunehmenden Anzahl an Tabletten der jeweiligen Tablettensorten und Zuordnen jedes ermittelten Tabletten-Einnahmezeitpunktes zu einem Tablettenfach (2)
und das Abfüllen der ermittelten Tabletten in das jeweilige zugeordnete Tablettenfach umfasst.

5. Tablettenabfüllverfahren nach einem der vorhergehenden Ansprüche, **dadurch gekennzeichnet, dass** beim Empfangen des Patientenauftrages eine Plausibilitätskontrolle der Rezeptdaten (REZ) hinsichtlich möglicher Überdosierung und gegenseitiger Verträglichkeit von Tablettensorten durchgeführt und bei Erkennen von Überdosierung oder Unverträglichkeit der Patientenauftrag zurückgewiesen wird.

6. Tablettenabfüllverfahren nach einem der vorhergehenden Ansprüche, **dadurch gekennzeichnet, dass** die Tablettenbehälter (1) mit aus den Rezeptdaten (REZ) abgeleiteter Information, wie den Einnahmevorschriften und/oder der Patienten-Identifikation (PAT-ID), versehen werden, vorzugsweise durch Bedrucken oder Etikettieren des Tablettenbehälters (1).

7. Tablettenabfüllverfahren nach einem der vorhergehenden Ansprüche, **dadurch gekennzeichnet, dass** beim Auftreten von Fehlern bei der Durchführung des Tablettenabfüllverfahrens für einen Tablettenbehälter das Tablettenabfüllverfahren abgebrochen und der Tablettenbehälter ausgeschieden wird.

8. Tablettenabfüllverfahren nach Anspruch 7 in Verbindung mit einem der Ansprüche 2 oder 3, **dadurch gekennzeichnet, dass** beim Auftreten von Fehlern bei der Durchführung des Tablettenabfüllverfahrens für einen Tablettenbehälter Fehlerkennzeichnungen auf den diesem Tablettenbehälter zugeordneten Informationsträger (11) geschrieben werden und solcherart mit Fehlerkennzeichnungen gekennzeichnete Tablettenbehälter beim Abgeben ausgeschieden werden.

9. Tablettenabfüllverfahren nach Anspruch 8, **dadurch gekennzeichnet, dass** vor jedem Verarbeitungsschritt des Tablettenabfüllverfahrens für jeden Tablettenbehälter (1) überprüft wird, ob der diesem Tablettenbehälter zugeordnete Informationsträger (11) eine Fehlerkennzeichnung enthält und bei Erkennen einer solchen Fehlerkennzeichnung der jeweilige Verarbeitungsschritt nicht durchgeführt wird.

10. Tablettenabfüllverfahren nach einem der vorhergehenden Ansprüche, **dadurch gekennzeichnet, dass** das Abgeben der befüllten Tablettenbehälter das Sammeln aller zu einem Patientenauftrag gehörenden Tablettenbehälter umfasst.

11. System zum automatischen Abfüllen von Tabletten in Tablettenbehälter, die zumindest ein Tablettenfach zur Aufnahme von Tabletten aufweisen, **gekennzeichnet durch**
Empfangsmittel (30) zum Empfangen eines Patientenauftrages, der eine Patienten-Identifikation (PAT-ID) und zumindest ein Rezept mit Rezeptdaten (REZ) über von dem Patienten einzunehmende Tablettensorten und ihre Einnahmevorschriften enthält;
Zuordnungsmittel (40) zum Zuordnen der Rezeptdaten (REZ) und Patienten-Identifikation (PAT-ID) zu jeweiligen Tablettenbehältern (1);
zumindest einen Tablettenabgabeautomaten (50), der Vorräte an einer Vielzahl von Tablettensorten enthält, zum automatischen Befüllen zumindest eines Tablettenfaches eines jeweiligen Tablettenbehälters,
wobei die Zuordnungsmittel (40) oder der Tablettenabgabeautomat (50) dazu ausgebildet sind/ist, aus den Rezeptdaten (REZ) die von einem Patienten zu jeweiligen Zeitpunkten einzunehmende Anzahl an Tabletten der jeweiligen Tablettensorten zu ermitteln und jedem ermittelten Tabletten-Einnahmezeitpunkt ein Tablettenfach (2) eines Tablettenbehälters (1) zuzuordnen, und der Tablettenabgabeautomat (50) dazu ausgebildet ist, die so ermittelten Tabletten für jeden ermittelten Einnahmezeitpunkt in das zugeordnete Tablettenfach (2) des jeweiligen Tablettenbehälters (1) abzufüllen;
Verschließmittel (70) zum Verschließen der befüllten Tablettenbehälter (1).

12. Tablettenabfüllsystem nach Anspruch 11, **dadurch gekennzeichnet, dass** die Zuordnungsmittel (40) zum Übertragen der Rezeptdaten (REZ) und Patienten-Identifikation (PAT-ID) auf einen oder mehrere Informationsträger (11) und zum Zuordnen eines jeweiligen Informationsträgers (11) zu jeweils einem Tablettenbehälter (1) ausgebildet sind.

13. Tablettenabfüllsystem nach Anspruch 12, **dadurch gekennzeichnet, dass** der Informationsträger (11) ein, vorzugsweise berührungslos, beschreibbarer und auslesbarer elektronischer Datenträger, oder ein bedruckbares Substrat, wie ein Barcodeetikett, ist.

14. Tablettenabfüllsystem nach Anspruch 12 oder 13, **dadurch gekennzeichnet, dass** der Tablettenabgabeautomat (50) zum Auslesen der auf dem Infomiationsträger (11) gespeicherten Rezeptdaten (REZ) und zum Abfüllen von Tabletten in Tablettenfächer (2) des dem Informationsträger (11) zugeordneten Tablettenbehälters (1) gemäß den aus den Rezeptdaten (REZ) ermittelbaren von dem Patienten zu jeweiligen Zeitpunkten einzunehmenden Anzahl an Tabletten der jeweiligen Tablettensorten ausgebildet ist.

15. Tablettenabfüllsystem nach einem der Ansprüche 11 bis 14, **dadurch gekennzeichnet, dass** für jeden Tablettenbehälter (1) eine Trägereinrichtung (10) vorgesehen ist, auf der der Tablettenbehälter durch das Tablettenabfüllsystem transportierbar ist.

16. Tablettenabfüllsystem nach Anspruch 15 in Verbindung mit Anspruch 12 oder 13, **dadurch gekennzeichnet, dass** der Informationsträger (11) auf der Trägereinrichtung (10) angeordnet ist.

17. Tablettenabfüllsystem nach einem der Ansprüche 11 bis 16, **dadurch gekennzeichnet, dass** jeder Tablettcnautomat (50) eine Vielzahl von Tablettenabgabestationen (51) umfasst, die jeweils einen Vorrat einer Tablettensorte enthalten und zur Abgabe einer einstellbaren Anzahl von Tabletten an beliebige Tablettenfächer (2) der Tablettenbehälter (1) ausgebildet sind.

18. Tablettenabfüllsystem nach einem der Ansprüche 11 bis 17, **dadurch gekennzeichnet, dass** die Empfangsmittel (30) dazu ausgebildet sind, die Rezeptdaten (REZ) hinsichtlich möglicher Überdosierung und gegenseitiger Verträglichkeit von Tablettensorten zu überprüfen und bei Erkennen von Überdosierung oder Unverträglichkeit den Patientenauftrag zurückzuweisen.

19. Tablettenabfüllsystem nach einem der Ansprüche 11 bis 18, **gekennzeichnet durch** Druck- oder Etikettiermittel (80) zum Versehen der Tablettenbehälter (1) mit aus den Rezeptdaten (REZ) abgeleiteter Information, wie den Einnahmevorschriften und/oder der Patienten-Identifikation (PAT-ID).

20. Tablettenabfüllsystem nach Anspruch 12 oder 13, wobei die Informationsträger (11) zum Empfangen von Fehlerkennzeichnungen ausgebildet sind, **dadurch gekennzeichnet, dass** Kontrollmittel (60) vorgesehen sind, die dazu ausgebildet sind, die Fehlerkennzeichnungen von den Informationsträgern (11) zu lesen und mit Fehlerkennzeichnungen gekennzeichnete Tablettenbehälter (1) auszuscheiden.

21. Tablettenabfüllsystem nach einem der Ansprüche 11 bis 20, **dadurch gekennzeichnet, dass** Sammelmittel (90) zum Sammeln aller zu einem Patientenauftrag gehörenden Tablettenbehälter (1) vorgesehen sind.

22. Tablettenabfüllsystem nach einem der Ansprüche 11 bis 21, **dadurch gekennzeichnet, dass** jede Tablettenabgabestation (51) eine Vielzahl von Tablettenabgabeeinheiten (54) umfasst, die von einem gemeinsamen Tablettenmagazin (53) versorgt werden, wobei die Tablettenabgabeeinheiten (54) vorzugsweise als Rotationsabgabeeinheiten ausgebildet sind.

23. Tablettenabfüllsystem nach Anspruch 22, **dadurch gekennzeichnet, dass** das Tablettenmagazin (53) mit einem auswechselbaren Pufferbehälter (52) verbindbar ist.

## Claims

1. A method for automatically filling tablets into tablet containers comprising at least one tablet compartment for receiving tablets, **characterized by**:
receiving a patient order containing a patient identification (PAT-ID) and at least one prescription comprising prescription data (REZ) regarding the types of tablets to be taken by the patient and instructions for taking said tablets;
allocating the prescription data (REZ) and patient identification (PAT-ID) to respective tablet containers (1);
automatically filling at least one tablet compartment (2) of a respective tablet container (1) by means of at least one automatic tablet dispenser (50) containing supplies of a plurality of tablet types,
wherein the number of tablets of the respective types of tablets to be taken by a patient at certain times is determined from the prescription data (REZ) and a tablet compartment (2) of a tablet container (1) is allocated to each determined time for taking the tablets and the tablets thus determined are filled for each determined time for taking them into the respective tablet compartment (2) that has been allocated;
sealing and delivering the filled tablet containers (1).

2. A tablet filling method according to claim 1, **characterized in that** the allocation of prescription data (REZ) and patient identification (PAT-ID) to respective tablet containers (1) comprises the transmission of prescription data and patient identification to one or several information carriers (11) and the allocation of a respective information carrier to one tablet container (1) at a time.

3. A tablet filling method according to claim 2, **characterized in that** an electronic data carrier, which preferably is writable and readable in a contactless manner, or a printable substrate such as a bar-code label is provided as an information carrier (11).

4. A tablet filling method according to claim 2 or 3, **characterized in that** the automatic filling of at least one tablet compartment (2) of a respective tablet container (1) by at least one automatic tablet dispenser (50) comprises conveying the tablet containers (1) and the information carriers (11) allocated thereto jointly through the at least one automatic tablet dispenser;
reading out the prescription data stored on the information carrier (11) by the respective automatic tablet dispenser (50);
checking by means of the respective automatic tablet dispenser (50) as to whether the supplies of tablet types stored in it correspond to one of the tablet types contained in the prescription data (REZ) and, in case of correspondence,
determining the number of tablets of the respective types of tablets to be taken by the patient at certain times and allocating a tablet compartment (2) to each determined time for taking the tablets,
and filling the determined tablets into the respective tablet compartment that has been allocated.

5. A tablet filling method according to any of the preceding claims, **characterized in that**, upon receiving the patient order, a plausibility check of the prescription data (REZ) regarding a possible overdosage and the mutual compatibility between tablet types is carried out and, in case an overdosage or an incompatibility is detected, the patient order is rejected.

6. A tablet filling method according to any of the preceding claims, **characterized in that** the tablet containers (1) are provided with information derived from the prescription data (REZ), such as instructions for taking the tablets, and/or the patient identification (PAT-ID), preferably by imprinting or labelling the tablet container (1).

7. A tablet filling method according to any of the preceding claims, **characterized in that**, if errors occur during the implementation of the tablet filling process for a tablet container, the tablet filling process is stopped and the tablet container is eliminated.

8. A tablet filling method according to claim 7 in connection with any of claims 2 or 3, **characterized in that**, if errors occur during the implementation of the tablet filling process for a tablet container, error identifications are written onto the information carrier (11) allocated to said tablet container and tablet containers marked with error identifications in this way are eliminated during the delivery.

9. A tablet filling method according to claim 8, **characterized in that**, prior to each processing step of the tablet filling process, it is checked for each tablet container (1) as to whether the information carrier (11) allocated to said tablet container contains an error identification and, upon detection of such an error identification, the respective processing step is not carried out.

10. A tablet filling method according to any of the preceding claims, **characterized in that** the delivery of the filled tablet containers comprises collecting all tablet containers belonging to a respective patient order.

11. A system for automatically filling tablets into tablet containers comprising at least one tablet compartment for receiving tablets, **characterized by**:
reception means (30) for receiving a patient order containing a patient identification (PAT-ID) and at least one prescription comprising prescription data (REZ) regarding the types of tablets to be taken by the patient and instructions for taking said tablets;
allocation means (40) for allocating the prescription data (REZ) and patient identification (PAT-ID) to respective tablet containers (1);
at least one automatic tablet dispenser (50) containing supplies of a plurality of tablet types for automatically filling at least one tablet compartment of a respective tablet container, wherein the allocation means (40) or the automatic tablet dispenser (50) is/are designed for determining, from the prescription data (REZ), the number of tablets of the respective types of tablets to be taken by a patient at certain times and for allocating a tablet compartment (2) of a tablet container (1) to each determined time for taking the tablets, and the automatic tablet dispenser (50) is designed for filling the tablets thus determined for each determined time for taking them into the tablet compartment (2) of the respective tablet container (1), which compartment has been allocated;
closing means (70) for sealing the filled tablet containers (1).

12. A tablet filling system according to claim 11, **characterized in that** the allocation means (40) are designed for transmitting the prescription data (REZ) and patient identification (PAT-ID) to one or several information carriers (11) and for allocating a respective information carrier (11) to one tablet container (1) at a time.

13. A tablet filling system according to claim 12, **characterized in that** the information carrier (11) is an electronic data carrier, which preferably is writable and readable in a contactless manner, or a printable substrate such as a bar-code label.

14. A tablet filling system according to claim 12 or 13, **characterized in that** the automatic tablet dispenser (50) is designed for reading out the prescription data (REZ) stored on the information carrier (11) and for filling tablets into tablet compartments (2) of the tablet container (1) allocated to the information carrier (11) according to the number of tablets of the respective types of tablets to be taken by the patient at certain times, which number is determinable from the prescription data (REZ).

15. A tablet filling system according to any of claims 11 to 14, **characterized in that** for each tablet container (1) a support facility (10) is provided on which the tablet container is conveyable through the tablet filling system.

16. A tablet filling system according to claim 15 in connection with claim 12 or 13, **characterized in that** the information carrier (11) is placed on the support facility (10).

17. A tablet filling system according to any of claims 11 to 16, **characterized in that** each automatic tablet dispenser (50) comprises a plurality of tablet dispensing stations (51), each containing a supply of a tablet type and being designed for dispensing an adjustable number of tablets to random tablet compartments (2) of the tablet containers (1).

18. A tablet filling system according to any of claims 11 to 17, **characterized in that** the reception means (30) are designed for checking the prescription data (REZ) for a possible overdosage and the mutual compatibility between tablet types and for rejecting the patient order in case an overdosage or an incompatibility is detected.

19. A tablet filling system according to any of claims 11 to 18, **characterized by** printing or labelling means (80) for providing the tablet containers (1) with information derived from the prescription data (REZ), such as instructions for taking the tablets, and/or the patient identification (PAT-ID).

20. A tablet filling system according to claim 12 or 13, wherein the information carriers (11) are designed for receiving error identifications, **characterized in that** control means (60) are provided which are designed for reading the error identifications from the information carriers (11) and eliminating tablet containers (1) marked with error identifications.

21. A tablet filling system according to any of claims 11 to 20, **characterized in that** collecting means (90) are provided for collecting all tablet containers (1) belonging to a respective patient order.

22. A tablet filling system according to any of claims 11 to 21, **characterized in that** each tablet dispensing station (51) comprises a plurality of tablet dispensing units (54) supplied by a common tablet magazine (53), with the tablet dispensing units (54) preferably being designed as rotary dispensing units.

23. A tablet filling system according to claim 22, **characterized in that** the tablet magazine (53) is connectable to a replaceable buffer container (52).

## Revendications

1. Procédé pour le remplissage automatique de comprimés dans des récipients à comprimés qui comportent au moins un casier à comprimés pour recevoir des comprimés, **caractérisé par** les étapes suivantes :
réception d'une commande d'un patient, qui contient une identification de patient (PAT-ID) et au moins une ordonnance avec des données d'ordonnance (REZ) concernant les sortes de comprimés à prendre par le patient et leurs posologies ;
association des données d'ordonnance (REZ) et de l'identification du patient (PAT-ID) à des récipients à comprimés (1) respectifs ;
remplissage automatique d'au moins un casier à comprimés (2) d'un récipient à comprimés (1) respectif par au moins un appareil automatique de distribution de comprimés (50) qui contient des réserves d'une pluralité de sortes de comprimés,
dans lequel on détermine à partir des données d'ordonnance (REZ) le nombre de comprimés, de la sorte de comprimés respective, qui doivent être pris par un patient au moment respectif, et chaque moment déterminé pour la prise d'un comprimé est associé à un casier à comprimés (2) d'un récipient à comprimés (1), et les comprimés ainsi déterminés sont remplis pour chaque moment de prise déterminé dans le casier à comprimés associé (2) ; et
fermeture et distribution des récipients à comprimés remplis (1).

2. Procédé de remplissage de comprimés selon la revendication 1, **caractérisé en ce que** l'association des données d'ordonnance (REZ) et de l'identification du patient (PAT-ID) à des récipients à comprimés respectifs (1) comprend la transmission des données d'ordonnance et de l'identification du patient vers un ou plusieurs supports d'information (11), et l'association d'un support d'information respectif à un récipient à comprimés respectif (1).

3. Procédé de remplissage de comprimés selon la revendication 2, **caractérisé en ce que** l'on prépare à titre de support d'information (11) un support de données électronique inscriptible et lisible, de préférence sans contact, ou un substrat imprimable, comme une étiquette avec code à barres.

4. Procédé de remplissage de comprimés selon la revendication 2 ou 3, **caractérisé en ce que** le remplissage automatique d'au moins un casier à comprimés (2) d'un récipient à comprimés respectif (1) par au moins un appareil automatique de distribution de comprimés (50) comprend :
la lecture des données d'ordonnance mémorisées sur le support d'information (11) par l'appareil automatique de distribution de comprimés (50) respectif ;
la vérification par l'appareil automatique de distribution de comprimés (50) respectif si les réserves stockées dans celui-ci de sortes de comprimés correspondent à l'une des sortes de comprimés contenues dans les données d'ordonnance (REZ) et, en cas de correspondance la détermination du nombre de comprimés, des sortes de comprimés respectifs, que le patient doit prendre à des moments respectifs et l'association de chaque moment de prise de comprimés déterminé à un casier à comprimés (2), et
le remplissage des comprimés déterminés dans le casier à comprimés associé respectif.

5. Procédé de remplissage de comprimés selon l'une des revendications précédentes, **caractérisé en ce que** lors de la réception de la commande d'un patient on exécute un contrôle de plausibilité des données d'ordonnance (REZ) à l'égard d'un surdosage éventuel et de la compatibilité réciproque de sortes de comprimés, et la commande du patient est refusée en cas de reconnaissance d'un surdosage ou d'une incompatibilité.

6. Procédé de remplissage de comprimés selon l'une des revendications précédentes, **caractérisé en ce que** les récipients à comprimés (1) sont dotés d'informations dérivées des données d'ordonnance (REZ), comme la posologie et/ou l'identification du patient (PAT-ID), par exemple par impression ou par étiquetage du récipient à comprimés (1).

7. Procédé de remplissage de comprimés selon l'une des revendications précédentes, **caractérisé en ce que**, en cas d'apparition d'erreurs lors de l'exécution du procédé de remplissage de comprimés pour un récipient à comprimés, le procédé de remplissage de comprimés est interrompu et le récipient à comprimés est enlevé.

8. Procédé de remplissage de comprimés selon la revendication 7 en association avec l'une des revendications 2 ou 3, **caractérisé en ce que**, en cas d'apparition d'erreurs lors de l'exécution du procédé de remplissage de comprimés pour un récipient à comprimés, on inscrit des caractéristiques d'erreur sur le support d'information (11) associé à ce récipient à comprimés, et les récipients à comprimés caractérisés de telle façon avec des caractéristiques d'erreur sont enlevés lors de la distribution.

9. Procédé de remplissage de comprimés selon la revendication 8, **caractérisé en ce qu'**avant chaque étape du procédé de remplissage de comprimés on vérifie pour chaque récipient à comprimés (1) si le support d'information (11) associé à ce récipient à comprimés contient une caractéristique d'erreur et, en cas de reconnaissance d'une telle caractéristique erreur, l'étape respective n'est pas exécutée.

10. Procédé de remplissage de comprimés selon l'une des revendications précédentes, **caractérisé en ce que** la distribution des récipients à comprimés remplis inclut la collecte de tous les récipients à comprimés appartenant à une commande d'un patient.

11. Système pour le remplissage automatique de comprimés dans des récipients à comprimés, qui comprennent au moins un casier à comprimés pour la réception de comprimés, **caractérisé par** :
des moyens de réception (30) pour recevoir une commande d'un patient qui contient une identification du patient (PAT-ID) et au moins une ordonnance avec des données d'ordonnance (REZ) concernant les sortes de comprimés à prendre par le patient et leurs posologies ;
des moyens d'association (40) pour associer les données d'ordonnance (REZ) et l'identification du patient (PAT-ID) à des récipients à comprimés (1) respectifs ;
au moins un appareil automatique de distribution de comprimés (50), qui contient des réserves d'une pluralité de sortes de comprimés, pour le remplissage automatique d'au moins un casier à comprimés d'un récipient à comprimés respectif,
dans lequel les moyens d'association (40) ou l'appareil automatique de distribution de comprimés (50) est/sont réalisé(s) pour déterminer, à partir des données d'ordonnance (REZ), le nombre de comprimés, des sortes de comprimés respectifs, que le patient doit prendre à des moments respectifs, et pour associer à chaque moment de prise de comprimés déterminés un casier à comprimés (2) d'un récipient à comprimés (1), et l'appareil automatique de distribution de comprimés (50) est réalisé pour remplir les comprimés ainsi déterminés pour chaque moment de prise déterminé dans le casier à comprimés associé (2) du récipient à comprimés respectif (1) ; et
des moyens de fermeture (70) pour fermer les récipients à comprimés (1) remplis.

12. Système de remplissage de comprimés selon la revendication 11, **caractérisé en ce que** les moyens d'association (40) sont réalisés pour transmettre les données d'ordonnance (REZ) et l'identification du patient (PAT-ID) vers un ou plusieurs supports d'information (11), et pour associer un support d'information respectif (11) à un récipient à comprimés respectif (1).

13. Système de remplissage de comprimés selon la revendication 12, **caractérisé en ce que** le support d'information (11) est un support de données électronique inscriptible et lisible, de préférence sans contact, ou un substrat imprimable, comme une étiquette avec codes à barres.

14. Système de remplissage de comprimés selon la revendication 12 ou 13, **caractérisé en ce que** l'appareil automatique de distribution de comprimés (50) est réalisé pour la lecture des données d'ordonnance (REZ) mémorisées sur le support d'information (11) et pour le remplissage de comprimés dans des casiers à comprimés (2) du récipient à comprimés (1) associé au support d'information (11) conformément au nombre de comprimés, des sortes de comprimés respectifs, susceptible d'être déterminé à partir des données d'ordonnance (REZ), que le patient doit prendre aux moments respectifs.

15. Système de remplissage de comprimés selon l'une des revendications 11 à 14, **caractérisé en ce que** pour chaque récipient à comprimés (1), il est prévu un dispositif porteur (10) sur lequel le récipient à comprimés peut être transporté à travers le système de remplissage de comprimés.

16. Système de remplissage de comprimés selon la revendication 15 en association avec la revendication 12 ou 13, **caractérisé en ce que** le support d'information (11) est agencé sur le dispositif porteur (10).

17. Système de remplissage de comprimer selon l'une des revendications 11 à 16, **caractérisé en ce que** chaque appareil automatique de distribution de comprimés (50) comprend une pluralité de stations de distribution de comprimés (51), qui contiennent chacune une réserve d'une sorte de comprimés et qui sont réalisées pour la distribution d'un nombre réglable de comprimés vers des casiers à comprimés quelconques (2) des récipients à comprimés (1).

18. Système de remplissage de comprimés selon l'une des revendications 11 à 17, **caractérisé en ce que** les moyens de réception (50) sont réalisés pour vérifier les données d'ordonnance (REZ) à l'égard d'un surdosage éventuel et d'une compatibilité réciproque de sortes de comprimés, et pour refuser la commande du patient en cas de reconnaissance d'un surdosage ou d'une incompatibilité.

19. Système de remplissage de comprimés selon l'une des revendications 11 à 18, **caractérisé par** des moyens d'impression ou d'étiquetage (80) pour doter le récipient à comprimés (1) d'informations dérivées des données d'ordonnance (REZ), comme la posologie et/ou l'identification du patient (PAT-ID).

20. Système de remplissage de comprimés selon la revendication 12 ou 13, dans lequel les supports d'information (11) sont réalisés pour la réception de caractéristiques d'erreur, **caractérisé en ce qu'**il est prévu des moyens de contrôle (60) qui sont réalisés pour lire les caractéristiques d'erreur depuis les supports d'information (11), et pour enlever des récipients à comprimés (1) caractérisés avec des caractéristiques d'erreur.

21. Système de remplissage de comprimés selon l'une des revendications 11 à 20, **caractérisé en ce qu'**il est prévu des moyens de collecte (90) pour collecter tous les récipients à comprimés (1) appartenant à une commande d'un patient.

22. Système de remplissage de comprimés selon l'une des revendications 11 à 21, **caractérisé en ce que** chaque station de distribution de comprimés (51) comprend une pluralité d'unités de distribution de comprimés (54) qui sont alimentées depuis un magasin à comprimés (53) commun, les unités de distribution de comprimés (54) étant de préférence réalisées sous forme d'unités de distribution rotatives.

23. Système de remplissage de comprimés selon la revendication 22, **caractérisé en ce que** le magasin à comprimés (53) est susceptible d'être relié à un récipient tampon (52) interchangeable.
